## Europäisches Patentamt

## European Patent Office

## Office européen des brevets

(19)

(11) Veröffentlichungsnummer: **0 120 835**
**B1**

(12) **EUROPÄISCHE PATENTSCHRIFT**

(45) Veröffentlichungstag der Patentschrift: **01.08.90**

(21) Anmeldenummer: **84890044.5**

(22) Anmeldetag: **12.03.84**

(51) Int. Cl.$^5$: **A 61 K 35/16**, A 61 K 39/395

(54) **Verfahren zur Inaktivierung von Unverträglichkeitsreaktionen verursachenden Substanzen.**

(30) Priorität: **16.03.83 AT 931/83**

(43) Veröffentlichungstag der Anmeldung:
**03.10.84 Patentblatt 84/40**

(45) Bekanntmachung des Hinweises auf die
Patenterteilung:
**01.08.90 Patentblatt 90/31**

(84) Benannte Vertragsstaaten:
**AT BE CH DE FR GB IT LI NL SE**

(56) Entgegenhaltungen:
**DE-A-2 218 148**
**DE-A-2 936 047**
**DE-A-3 220 309**
**DE-B-1 148 037**

(73) Patentinhaber: IMMUNO Aktiengesellschaft für
chemisch-medizinische Produkte
Industriestrasse 72
A-1220 Wien (AT)

(72) Erfinder: **Eibl, Johann, Dr.**
**Gustav Tschermakgasse 2**
**A-1180 Wien (AT)**
Erfinder: **Linnau, Yendra, Dr.**
**Lavendlweg 24**
**A-1224 Wien (AT)**
Erfinder: **Schwarz, Otto, Dr.**
**Celtesgasse 5**
**A-1190 Wien (AT)**

(74) Vertreter: **Wolfram, Gustav, Dipl.-Ing.**
**Schwindgasse 7 P.O. Box 205**
**A-1041 Wien (AT)**

**Beschreibung**

Die Erfindung betrifft ein Verfahren zur Inaktivierung von Unverträglichkeitsreaktionen verursachenden Substanzen in therapeutisch oder prophylaktisch anzuwendenden immunglobulinhältigen Blutfraktionen unter Verwendung von proteolytisch wirksamen Enzymen.

Im besonderen betrifft die Erfindung ein Verfahren zur Inaktivierung solcher Substanzen bei der Herstellung von neuen Immunglobulin-G-hältigen, zur intravenösen Anwendung geeigneten Fraktionen aus menschlichem oder tierischem Plasma.

Immunglobulinhältige Präparate können bei primären und sekundären Immundefekten, A- oder Hypogammaglobulinamie, bei Antikörpermangelsyndrom, bei Virusinfektionen oder bakteriellen Infektionen angewendet werden.

Zur Gewinnung von immunglobulinhältigen Präparationen aus menschlichem oder tierischem Plasma sind bereits verschiedene Methoden bekannt, so z.B. die Ausfällung mit Äthanol (J. L. Oncley, M. Melin, D. A. Richart, J. W. Cameron und P. M. Gross, J. Am. Chem. Soc. 71, 541 (1949)) sowie modifizierte Äthanolverfahren nach H. F. Deutsch, L. J. Gosting, R. A. Alberty und J. W. Williams, J. Biol. Chem. 164, 109 (1964), sowie P. Kistler und H. Nitschmann, Vox Sanguinis 7, 414 (1962).

Weiters ist eine Methode bekannt, nach der Immunglobulin aus Plasma mittels Ammonsulfat und Polyäthylenglykol gefällt wird (A. Polson, G. M. Potgieter, J. F. Largrier, G. E. F. Mears und F. J. Jourbet, Biochim. Biophys. Acta. 82, 463 (1964)). Nach anderen Verfahren wurde die Anwendung von Ionenaustauschern vorgeschlagen (E. A. Peterson und H. A. Sober, J. Am. Chem. Soc. 78, 751 (1956)).

Diese Methoden hatten den Nachteil, daß die gewonnenen Präparate lediglich für eine intramuskuläre Applikation geeignet waren. Bei intravenöser Anwendung zeigten sie unerwünschte Nebenreaktionen, wie eine vasoaktive Wirkung.

Man hat sich daher bemüht, Nebenerscheinungen bzw. Nebenwirkungen zu verringern, zu welchem Zweck immunglobulinhältige Präparate mit löslichen proteolytischen Enzymen, wie Pepsin, Plasmin, Papain u.a. behandelt wurden (DE—PS 1 148 037 sowie CH—PS 392.780). Bei dieser Behandlung wird jedoch die Molekülstruktur der Immunglobuline verändert, was eine verkürzte biologische Halbwertszeit bzw. unerwünschte Nebenwirkungen, wie eine vasoaktive und leukopenische Aktivität zur Folge haben kann. Es wurde auch festgestellt, daß Enzymreste in den Präparaten zurückbleiben, wodurch diese kontaminiert werden. Die Lagerstabilität ist dementsprechend gering, die Gefahr des Fortschreitens der proteolytischen Spaltung groß.

In der DE—OS 28 46 412 ist ein Verfahren zur Herstellung von in ihrem Fc-Teil immunologisch veränderten Immunglobulinen beschrieben, wobei die immunglobulinhältige Fraktion mit einer Proteaselösung behandelt wird. Diese Produkte sollen bei lokaler oder systemischer Applikation eine antiallergene Wirkung ausüben. Für die Herstellung einer intravenös applizierbaren Präparation ist das Verfahren ungeeignet, weil die Proteaselösung aus dem Produkt nicht oder nicht vollständig entfernbar ist und im neutralen pH-Bereich seine Abbauwirkung fortsetzt.

In der DE—OS 29 36 047 ist ein Verfahren zur Herstellung eines intravenös verabreichbaren Immunglobulinpräparates beschrieben, bei welchem eine kombinierte Reinigung mit Ammoniumsulfat und Polyäthylenglykol in Gegenwart eines löslichen Kohlehydrates oder eines Polyols vorgenommen wird. Wenngleich damit vasoaktive Nebenerscheinungen vermieden werden konnten, so bleibt eine Verbesserung in bezug auf die Sicherheit und Reproduzierbarkeit bei intravenöser Anwendung weiterhin wünschenswert.

Zum Stand der Technik sind auch die japanischen Patentschriften JP—OS 56—7721 und JP—OS 56—15215 sowie die DE—OS 32 20 309 zu zählen, die Verfahren zur Herstellung von intravenös applizierbaren Immunglobulin-Präparaten zum Ziel haben. Die Behandlung soll mit immobilisiertem Plasmin bzw. immobilisiertem Pepsin erfolgen, doch sind auch die hiermit erreichbaren Ergebnisse nicht zufriedenstellend, weil die Präparate eine unerwünscht hohe antikomplementäre Aktivität aufweisen.

Die Erfindung bezweckt die Vermeidung der geschilderten Nachteile und Schwierigkeiten und stellt sich die Aufgabe, ein Verfahren zu schaffen, bei dem Unverträglichkeitsreaktionen verursachende Substanzen zuverlässig eliminiert werden und eine weitgehende Sicherheit der therapeutisch oder prophylaktisch anzuwendenden Präparationen gewährleistet wird.

Diese Aufgabe wird erfindungsgemäß dadurch gelöst, daß eine aus menschlichem oder tierischem Blut gewonnene immunglobulinhältige Blutfraktion mit an wasserunlösliches Trägermaterial gebundenen Pankreasenzymen, wie Trypsin oder Chymotrypsin oder Pankreasprotease, behandelt wird, um enthaltene unerwünschte Substanzen zu inaktivieren, das immobilisierte Enzym abgetrennt und die behandelte Fraktion gegebenenfalls einer weiteren Fraktionierung und Konzentrierung unterworfen wird.

Hierbei wird vorteilhaft als wasserunlösliches Trägermaterial Sepharose 4 B-Gel® verwendet.

Das gereinigte Immunglobulin kann aus der mit an wasserunlösliches Trägermaterial gebundenen Enzymen behandelten immunglobulinhältigen Fraktion, gegebenenfalls nach Entfernen unerwünschter Begleitstoffe, mit Proteinfällungsmitteln ausgefällt und zu einem Endprodukt verarbeitet werden.

Im besonderen hat sich eine Kombination der folgenden Reinigungs- und Konzentrationsmaßnahmen bewährt:

daß aus menschlichem oder tierischem Plasma durch Behandeln mit Äthanol bei einer Temperatur von unter 0°C ein immunglobulinhältiger Niederschlag ausgefällt wird;

daß der Niederschlag mittels einer Pufferlösung extrahiert und aus der erhaltenen Lösung durch neuerliche Behandlung mit Äthanol ein pastenförmiges Immunglobulinkonzentrat gewonnen wird;

daß dieses Konzentrat durch Dialyse gereinigt wird;

daß die so gereinigte immunglobulinhältige Fraktion mit einem immobilisierten Enzym aus der Gruppe Trypsin, Chymotrypsin oder Pankreasprotease bei erhöhter Temperatur von etwa 37°C behandelt und sodann das immobilisierte Enzym abgetrennt wird;

daß aus der so behandelten Fraktion gereinigtes, im wesentlichen aus, IgG bestehendes Immunglobulin mittels eines Proteinfällungsmittels, vorzugsweise Polyäthylenglykol, ausgefällt wird und

daß die Fällung gelöst, die Lösung sterilfiltriert und schließlich lyophilisiert wird.

Eine Analyse der molekularen Feinstruktur der erfindungsgemäß hergestellten Fraktionen ergibt eine Zusammensetzung von mindestens 90% monomeren IgG-Molekülen und mindestens 90% funktionell intakten IgG-Molekülen.

Die Bestimmung der monomeren IgG-Moleküle erfolgt durch Gelpermeationschromatographie (Gelfiltration) nach H. Determann, "Gel-Chromatographie", Springer-Verlag, Berlin, 1968, in folgender Weise:

Die Moleküle werden nach ihrem Molekulargewicht aufgetrennt. Moleküle, die größer sind als die größten Poren im gequollenen Gel, können nicht in das Gel eindringen und werden zuerst eluiert (das dazugehörige Elutionsvolumen heißt $V_o$). Kleinere Moleküle dringen in die Gelporen und wandern demzufolge langsamer (das dazugehörige Elutionsvolumen heißt $V_e$). Somit ist das Elutionsvolumen ($V_e$) ein charakteristischer Parameter eines Stoffes. Das relative Elutionsvolumen ($V_e/V_o$ eines Stoffes ist von den geometrischen Säulenabmessungen und der Säulenfüllung unabhängig. Die Bestimmung wird durchgeführt, indem z.B. eine Trennsäule von 2,6 cm Durchmesser und 100 cm Länge mit einem Gel, z.B. Agarose Polyacrylamid (Handelsname Ultrogel AcA 34)® gefüllt wird, das in einem Natriumphosphat-Natriumchlorid-Puffer (PBS), pH 7,0, gequollen wurde. 50 mg eines Immunglobulinpräparates werden auf der Säule aufgetragen und mit Natriumphosphat-Natriumchlorid-Puffer, pH 7,0, mit einer Durchflußgeschwindigkeit von 20 ml/h eluiert.

Die Eluate werden zu 4,5 ml-Fraktionen aufgefangen und die Elutionskurve wird mittels eine UV-Detektors bei 280 nm ermittelt. Anhand des Elutionsdiagrammes werden die einzelnen Komponenten vereinigt und das Elutionsvolumen sowie die Proteinkonzentration bestimmt.

Die Immunglobuline mit einem relativen Elutionsvolumen $V_e/V_o$ zwischen 1,30 und 2,20 werden als Monomer IgG-Moleküle bezeichnet und betragen erfindungsgemäß mindestens 90% des gesamaten Proteins. In diesem Zusammenhang sei bemerkt, daß die IgG-Moleküle mit einem $V_e/V_o$ von 1,30 bis 1,65 als dimere IgG bezeichnet werden; da sie aber mit dem Monomer IgG-Molekül mit einem $V_e/V_o$ von 1,66 bis 2,20 in einem reversiblen Gleichgewicht zueinander stehen, sind sie als Monomere zu rechnen (siehe J. S. Finlayson, B. L. Armstrong und A. M. Young, Acta Radiologica Supplementum *310*, (1971), 114).

Die Bestimmung der funktionell intakten IgG-Moleküle erfolgt nach der Protein-A-Sepharose Methode (FEBS Letters, Vol. 28, 1972, 73 ff.; H. Hjelm, K. Hjelm, J. Sjöquist, "Protein A from staphylococcus aureus, its solution by affinity chromatography and its use as an immunosorbent for isolation of immunolglobulins"). Die Methode beruht darauf, daß das Protein A des Staphylokokkus aureus mit den IgG-Molekülen der Subklassen IgG 1, 2 und 4 in Wechselwirkung tritt und sie bindet. Die funktionell aktiven Stellen sind die $C_H2$ und $C_H3$-Regionen, das sind Teile der H-Kette der IgG-Moleküle.

Die vereinigten Fraktionen $V_e/V_o$ von 1,30 bis 2,20 aus der Molekulargewichtsbestimmung mittels Gelfiltration werden auf eine bestimmte Proteinkonzentration eingestellt und 10 mg Protein dieser Präparation über 10 ml Protein A-Sepharose, immobilisiertes Protein A, chromatographiert. Die gebundenen IgG 1, 2 und 4 werden mit einem Natriumcitrat-Citronensäure-Puffer, pH 3,0, eluiert. Dann wird das gebundene und ungebundene IgG errechnet.

Nach einer bevorzugten Ausführungsform weist die erfindungsgemäß hergestellte Fraktion eine so geringe antikomplementäre Wirkung auf, daß sie nicht weniger als 40 mg Protein zur Neutralisation einer CH50-Einheit benötigt.

Die Bestimmung dieses Merkmales erfolgt gemäß "Public Health Monograph" Nr. 74; Standardized diagnostic complement fixation method and adaptation to microtest, Washington, 1965, und E. A. Kabal und M. Mayer, Experimental immunochemistry; 2nd ed. Thomas Springfield 1961.

Bei elektrophoretischer Bestimmung findet man bei den erfindungsgemäß hergestellten Fraktionen mindestens 95% Gammaglobulin. Die Bestimmung erfolgt gemäß Michael D. Gebott, Beckman Microzone Elektrophoresis Manual, Beckman Instruments, Inc. 1977, 015-083630-C.

Charakteristisch für die erfindungsgemäß hergestellten Immunglobulin-G-hältigen Fraktionen sind des weiteren ihre pharmakologischen Eigenschaften: sie sind nämlich im wesentlichen frei von vasoaktiv und leukopenisch wirksamen sowie von bronchospastischen Substanzen, ausgedrückt durch folgende Merkmale:

a) daß die vasoaktive Wirkung im Hundetest als Durchschnitt bei vier Tieren, d.h. ein Blutdruckabfall von höchstens 30%, erst bei einer Dosis von mehr als 500 mg/kg Körpermasse,

b) daß die leukopenische Wirkung im Hundetest als Durchschnitt bei vier Tieren, d.h. ein Abfall der Leukozytenzahl von höchstens 50%, erst bei einer Dosis von mehr als 500 mg/kg Körpermasse, und

c) daß die bronchospastische Wirkung im Meerschweinchentest als Durchschnitt bei vier Tieren, d.h.

ein Atemdruckanstieg von höchstens 30%, erst bei einer Dosis von mehr als 500 mg/kg Körpermasse feststellbar ist.

Die vasoaktive Wirkung wird in folgender Weise festgestellt:

Versuchstieren (Mischlingen beiderlei Geschlechts) wird nach Narkotisierung die Vena jugularis und die Arterie carotis präpariert. Vor der Anästhesie wird eine Fastenzeit von mindestens 12 Stunden angesetzt. Pro Testsubstanz werden je vier qualifizierte Hunde, d.h. solche Tiere benötigt, die bei intraarterieller Applizierung von standardisiertem intramuskulär anwendbarem Immunglobulin ("Standardsubstanz") eine vasoaktive Wirkung (Blutdruckabfall) von mindestens 30% bei einer Dosierung von 50 mg/kg Körpermasse zeigen. Dieses standardisierte intramuskulär anwendbare Immunglobulin wird nach der einleitend erwähnten Methode von J. L. Oncley, M. Melin, D. A. Richart, J. W. Cameron und P. M. Gross, J. Am. Chem. Soc., 71, 541 (1949) bereitet. Hunde, die auf die Standardsubstanz keine Reaktion zeigen, können für die Vergleichstests nicht herangezogen werden.

Von der Standardsubstanz werden 160 mg in 1 ml Aqua ad iniectabilia gelöst und mit isotoner NaCl-Lösung auf 16,7 mg/ml verdünnt. Das aufgelöste Material wird innerhalb von vier Stunden verwendet.

Das erfindungsgemäß hergestellte, intravenös anwendbare Immunglobulin-G wird mit Aqua ad iniectabilia so gelöst, daß 1 ml 165 mg Protein enthält. Das aufgelöste Material wird innerhalb von vier Stunden verwendet.

Die Tiere werden mit einer intravenösen Einzeldosis von 40 mg/kg Nembutal (Barbitural) anästhesiert, und die Vena jugularis externa wird nach ihrer Aufteilung am unteren Rand des Unterkiefers präpariert und ein Katheter eingebunden. Danach wird die Arterie carotis vom gleichen Hautschnitt aus freigelegt und ein Katheter eingebunden. Nach der Präparation der Arterie wird 30 min zugewartet, um stabile Ausgangswerte zu erreichen. Über den tiefen Venenkatheter wird der zentrale Venendruck und über den seichten arteriellen Katheter wird der arterielle Blutdruck mit Hilfe eines Drucktransducers gemessen. Über den arteriellen Katheter wird zuerst das erfindungsgemäß i.v. anwendbare Immunglobulin-G und dann die Standardsubstanz injiziert.

Während der gesamten Zeit der Versuchsdurchführung wird über den Arterienkatheter der systolische und diastolische Blutdruck mit Hilfe des Drucktransducers aufgezeichnet.

Der Blutdruckmittelwert (systolisch und diastolisch) sowie der Mittelwert der Leukozytenzahl von der Injektion der Testsubstanz und der Standardsubstanz werden bestimmt. Der maximale Blutdruckabfall wird durch Messung des Blutdruckes über 20 min nach Injektion der Testsubstanzen bestimmt.

Die vasoaktive Wirkung von erfindungsgemäß hergestelltem, iv. anwendbarem Immunglobulin-G wird so ermittelt, daß 500 mg/kg Körpermasse Hund injiziert werden und der durchschnittliche prozentuelle systolische und diastolische Blutdruckabfall bei vier Hunden mit der blutdrucksenden Wirkung von im. anwendbarer Standardsubstanz verglichen wird.

Die Bestimmung der leukopenischen Wirkung wird in folgender Weise durchgeführt:

Versuchstieren (Mischlingshunden beiderlei Geschlechts) wird nach Narkotisierung die Vena jugularis und die Arterie carotis präpariert. Vor der Anästhesie wird eine Fastenzeit von mindestens 12 Stunden angesetzt. Pro Testsubstanz werden je vier qualifizierte Hunde benötigt, die bei intraarterieller Applizierung von standardisiertem intramuskulär anwendbarem Immunglobulin (Standardsubstanz) eine leukopenische Wirkung (Leukozytenabfall) von mindestens 50% bei einer Dosierung von 50 mg/kg Körpermasse zeigen. Hunde, die auf die Standardsubstanz keine Reaktion zeigen, können für die Vergleichstests nicht herangezogen werden.

Die Vorbereitung der Versuchstiere und der Testsubstanzen erfolgt in gleicher Weise wie vorstehend beschrieben.

Zur Untersuchung der Leukozytenzahl werden Blutproben gezogen. Bei der ersten Blutprobe werden 40 myl Blut mit 20 ml Isotone II® (COULTER) und sechs Tropfen Zapoglobin® (COULTER) versetzt und im Coulter Counter gemessen. Anschließend werden nach 10, 15, 16, 17, 18 und 19 min weitere Blutproben zur Bestimmung der Leukozytenzahl gezogen. Dann werden sofort 500 mg Immunglobulin/kg Körpermasse iv. anwendbaren Immunglobulins-G innerhalb von 90 s intraarteriell injiziert. 1, 2, 3, 4, 5, 7, 10, 15 und 20 min nach Injektion werden weitere Blutproben gezogen. Nach 20 min werden 50 mg Immunglobulin/kg Körpermasse der Standardsubstanz innerhalb von 90 s injiziert. Blutproben werden wieder nach 1, 2, 3, 4, 5, 7, 10, 15 und 20 min gezogen.

Die maximale Senkung der Leukozytenzahl wird durch Bestimmung von Blutproben, die 1, 2, 3, 4, 5, 7, 10, 15 und 20 min nach der Injektion der Probe gezogen werden, bestimmt.

Die leukopenische Wirkung von iv. anwendbarem Immunglobulin-G wird so ermittelt, daß 500 mg/kg Körpermasse Hund injiziert werden und der durchschnittliche prozentuelle Leukozytenabfall bei vier Hunden mit der leukopenischen Wirkung von im. anwendbarer Standardsubstanz verglichen wird.

Die Bestimmung der bronchospastischen (atemdrucksteigernden) Wirkung beim Meerschweinchen erfolgt in folgender Weise:

Versuchstieren (Meerschweinchen männlichen Geschlechts) wird nach Narkotisierung die Luftröhre im Kehlkopfbereich präpariert. Nach Intubation wird das Versuchstier mittels eines Beatmungsgerätes mit einem der Körpermasse des Tieres entsprechenden Atemvolumen bei einer Atemfrequenz von 80/min beatmet. Danach wird die Arterie carotis vom gleichen Hautschnitt aus freipräpariert. Nach intraarterieller Injektion der Testsubstanz wird der Atemdruck laufend gemessen.

Für den Test werden laborgezüchtete Meerschweinchen männlichen Geschlechtes mit einem Körpergewicht zwischen 500 und 700 g verwendet. Pro Testsubstanz werden vier qualifizierte Meerschweinchen benötigt, die bei intraarterieller Applizierung von standardisiertem im. anwendbarem Immunglobulin (Standardsubstanz) einen Atemdruckanstieg um mindestens 30% bei einer Dosierung von 50 mg/kg Körpermasse zeigen. Meerschweinchen, die auf die Standardsubstanz keine Reaktion zeigen, können für die Vergleichstests nicht herangezogen werden.

Von der Standardsubstanz werden 160 mg in 1 ml Aqua ad iniectabilia gelöst und mit isotoner NaCl-Lösung auf 16,7 mg/ml verdünnt. Das aufgelöste Material wird innerhalb von vier Stunden verwendet.

Das erfindungsgemäß hergestellte iv. anwendbare Immunglobulin-G wird mit Aqua ad iniectabilia so gelöst, daß 1 ml 165 mg Protein enthält. Das aufgelöste Material wird innerhalb von vier Stunden verwendet.

Die Tiere werden narkotisiert; dann wird die Luftröhre im Kehlkopfbereich präpariert, und eine Trachealkanüle wird eingebunden. Mittels eines Beatmungsgerätes wird das Versuchstier mit einem der Körpermasse entsprechenden Atemvolumen bei einer Atemfrequenz von 80/min beatmet. Als Beatmungsgerät wird eine Harvard Pumpe Type 681 verwendet.

Danach wird die Arterie carotis vom gleichen Hautschnitt aus freipräpariert und ein Katheter eingebunden. Die Beatmungsdruckregistrierung erfolgt über einen Drucktransducer, der an den Beatmungsschlauch mit Hilfe eines T-Stückes angeschlossen ist.

Nach der Präparation wird zumindest 10 min gewartet, um stabile Ausgangswerte zu erreichen. Danach wird der Nullpunkt festgestellt und nach weiteren zwei bis drei Minuten 150 mg Immunglobulin/kg Körpermasse iv. anwendbares Immunglobulin-G innerhalb von 90 s intraarteriell über den Katheter injiziert.

Nach 20 min werden 50 mg Standardsubstanz/kg Körpermasse innerhalb von 90 s intraarteriell injiziert.

Der maximale Atemdruckanstieg während der auf die Injektion der Probe folgenden 20 min bestimmt und auf den Ausgangsmittelwert bezogen.

Die bronchospastische Wirkung von iv anwendbarem Immunglobulin-G wird so ermittelt, daß 500 mg/kg Körpermasse Meerschweinchen intraarteriell injiziert werden, und der durchschnittliche prozentuelle Atemdruck bei vier Meerschweinchen mit der atemdrucksteigernden Wirkung von im. anwendbarem Immunglobulin-G, Standardsubstanz, verglichen wird.

Die erfindungsgemäße Herstellung der Fraktionen wird durch die folgenden Arbeitsvorschriften und Beispiele näher erläutert:

Arbeitsvorschriften zur Bereitung eines immobilisierten Enzyms.

Arbeitsvorschrift 1:

1 l Sepharose 4 B-Gel® (Pharmacia) wird nach einer Waschung mit 4 l destilliertem Wasser mit 200 g Bromcyan, die in 100 ml Acetonitril gelöst wurden, bei einem pH-Wert von 11,0 versetzt. Das Reaktionsgemisch wird durch ein Eisbad gekühlt. Nach Entfernung der flüssigen Phase wird das Gel mit 800 mg Trypsin (Sigma), das in 1 l 0,2 molarem NaHCO₃ gelöst wurde, versetzt. Das nicht gebundene Trypsin wird vom Trypsin, das an das Gel gebunden ist, durch Filtrieren getrennt.

Nachdem das Gel-Trypsin mit 1 l einer 1 molaren Glycinlösung versetzt wurde, wird es gründlich mit 0,2 molarer NaHCO₃-Lösung proteinfrei gewaschen. Schließlich wird das Gel-Trypsin in 1 l 0,9 %iger NaCl-Lösung suspendiert — es ist gebrauchsfertig für die Inkubation mit einer Immunglobulinfraktion.

Arbeitsvorschrift 2:

Das unlösliche Enzym wird in der gleichen Weise wie in Arbeitsvorschrift 1 hergestellt, statt Trypsin wird Pankreasprotease (Merck) genommen.

Arbeitsvorschrift 3:

Arbeitsvorschrift 1 wird wiederholt, statt Trypsin wird Alpha-Chymotrypsin (Sigma) verwendet.

Beispiele zur Herstellung der Immunglobulin-G-hältigen Fraktion.

## Beispiel 1

Menschliches Blutplasma wird mit 8% Äthanol versetzt, bei einem pH-Wert von 7,2 und einer Temperatur von −2°C. Nach Abtrennen des Präzipitates wird die Äthanolkonzentration auf 25% erhöht und gleichzeitig die Temperatur auf −6°C gesenkt. Der ausfallende Niederschlag, der Immunglobulin enthält, wird weiter gereinigt durch eine Extraktion mit einem Phosphat-Acetat-Puffer und er wird mit 12% Äthanol bei einem pH-Wert von 5,4 und einer Temperatur von −2°C versetzt.

Der Niederschlag (der Alpha- und Betaglobulin enthält) wird verworfen. Die Äthanolkonzentration des Überstandes wird, bei einem pH-Wert von 7,2 und einer Temperatur von −10°C, auf 25% erhöht. Das ausgefallene, pastenförmige Immunglobulin wird gesammelt und das Äthanol durch Dialyse entfernt.

Danach wird zum Dialysat 170 g/l Ammoniumsulfat bei einem pH-Wert von 6,25 zugesetzt, das Präzipitat wird abgetrennt und verworfen. Zum Überstand wird bei einem pH-Wert von 7,2 weiteres Ammoniumsulfat bis zu einer Konzentration von 280 g/l zugefügt. Der Niederschlag wird in Wasser gelöst und zur Entfernung des Ammoniumsulfates dialysiert.

Nach der Dialyse wird die Ionenstärke der Immunglobulinlösung auf 0,15 gestellt.

100 g Immunglobulin werden mit 30 ml nach Arbeitsvorschrift 1 immobilisiertem Trypsin hergestellt und bei 37°C 72 Stunden lang behandelt. Nach Entfernung des Gel-Trypsins wird das behandelte Immuglobulin durch 135 g/l Polyäthylenglykol 4000 ausgefällt. Das Präzipitat wird in 0,9% NaCl aufgelöst, sterilfiltriert, abgefüllt und durch Gefriertrocknung haltbar gemacht.

Beispiel 2

Die Gewinnung der immunglobulinhältigen Fraktion erfolgt in gleicher Weise wie in Beispiel 1.

Die Inkubation wird mit der immobilisierten, nach Arbeitsvorschrift 2 hergestellten Pankreasprotease durchgeführt. 100 g Immunglobulin werden mit 70 ml gelförmiger immobilisierter Pankreasprotease behandelt und bei 37°C 70 Stunden gehalten. Nach Entfernung des Gels wird des Überstand mit 75 g/l Polyäthylenglykol 4000 versetzt und der Verunreinigungen enthaltende Niederschlag verworfen.

Zum Überstand wird weiteres Polyäthylenglykol 4000 bis zu einer Endkonzentration von 85 g/l zugesetzt. Der gebildete Niederschlag wird verworfen.

Durch Erhöhung der Polyäthylenkonzentration auf 135 g/l wird das gereinigte Immunglobulin ausgefällt und wie in Beispiel 1 haltbar gemacht.

Beispiel 3

Die Gewinnung der Immunglobulin-G-hältigen Fraktion wird in gleicher Weise, wie in Beispiel 1 beschrieben, vorgenommen, jedoch wird die Behandlung mit immobilisiertem Alpha-Chymotrypsin bei 37°C 72 Stunden durchgeführt.

Die Kennwerte der nach den Beispielen 1 bis 3 hergestellten erfindungsgemäßen Immunglobulin-G-hältigen Fraktionen, nämlich die Gehalte an monomeren IgG-Molekülen, die Gehalte an funktionell intakten IgG-Molekülen, die antikomplementäre Aktivität sowie der Gehalt an Gammaglobulin bei elektrophoretischer Auftrennung wurden, wie nachstehend beschrieben, bestimmt. Diese Werte sind in den folgenden Tabellen sowie in dem beiliegenden Diagramm I erläutert:

Das Diagramm I veranschaulicht eine Elutionskurve unter den angegebenen Bedingungen zwischen 150 und 400 ml sowie das relative Elutionsvolumen $V_e/V_o$. Die Kurve gibt den Proteingehalt der einzelnen Fraktionen wieder, gemessen bei einer Extinktion von 280 nm.

In der folgenden Tabelle 1 ist das $V_e/V_o$-Verhältnis nach der durchgeführten Gelpermeationschromatographie für die einzelnen, von der Kurve erfaßten Bereiche angegeben. Wie ersichtlich, ist das $V_e/V_o$-Verhältnis im Bereich von 1,30 bis 2,20 über 90%.

### Tabelle 1

### Gelpermeationschromatographie

| | $V_e/V_o$ | | |
|---|---|---|---|
| | 1,0 - 1,29 | 1,30 - 2,20 | 2,21 - 2,70 |
| Beispiel 1 | Spuren | 92,4 % | 7,6 % |
| Beispiel 2 | - | 93,2 % | 6,8 % |
| Beispiel 3 | - | 91,8 % | 8,2 % |

In der Tabelle 2 ist das Verhältnis der an Protein A gebundenen und der nichtgebundenen IgG-Moleküle dargestellt, wobei die gebundenen den funktionell intakten entsprechen. Wie ersichtlich, ist der Gehalt der funktionell intakten IgG-Moleküle in der gesamten Fraktion über 90%.

Tabelle 2

Affinitätschromatographie mit Protein A-Sepharose

| | % von Fraktion $V_e/V_o$ 1,30 - 2,30 | | % von gesamter Immun- globulin-G-hältiger Fraktion |
|---|---|---|---|
| | nichtgebunden | gebunden | |
| Beispiel 1 | 1,5 | 98,5 | 91,0 |
| Beispiel 2 | 0,9 | 99,1 | 92,3 |
| Beispiel 3 | 1,2 | 98,8 | 90,7 |

In Tabelle 3 sind die Werte für die antikomplementäre Aktivität und für die Elektrophorese angegeben, woraus ersichtlich ist, daß bei den Präparationen nach allen Beispielen Werte der antikomplementären Aktivität von mehr als 50 mg Immunglobulin-G-hältiger Fraktion zur Neutralisierung einer C'H-50-Einheit benötigt und elektrophoretisch bestimmte Werte an reinem Gammaglobulin von mehr als 97% erhalten wurden.

Tabelle 3

| | Antikomplementäre Aktivität | Elektrophorese |
|---|---|---|
| Beispiel 1 | > 50 mg/C'H-50 | >97,0 % reines Gammaglobulin |
| Beispiel 2 | > 50 mg/C'H-50 | > 97,0 % reines Gammaglobulin |
| Beispiel 3 | > 50 mg/C'H-50 | > 97,0 % reines Gammaglobulin |

Die pharmakologischen Merkmale bzw. Kennwerte der nach den Beispielen 1 bis 3 erfindungsgemäß hergestellten Immunglobulin-G-hältigen Fraktionen, nämlich die vasoaktive und leukopenische Wirkung im Hundetest und die bronchospastische Wirkung im Meerschweinchentest, wurden wie im Vorherstehenden beschrieben bestimmt; diese Werte sind aus den folgenden Tabellen zu ersehen:

TABELLE 4

Durchschnittsblutdruck von vier Hunden in % des Ausgangswertes nach Injizierung von 500 mg der erfindungsgemäß hergestellten Präparation pro kg Körpermasse

| | Systolischer Blutdruck | Diastolischer Blutdruck |
|---|---|---|
| Präparation nach Beispiel 1 | 91 % | 86 % |
| Präparation nach Beispiel 2 | 93 % | 89 % |
| Präparation nach Beispiel 3 | 81 % | 79 % |

# EP 0 120 835 B1

Tabelle 5

Durchschnittsleukozytenzahl von vier Hunden in % des Ausgangswertes nach Injizierung von 500 mg der erfindungsgemäß hergestellten Präparation pro kg Körpermasse

| | |
|---|---|
| Beispiel 1 | 73% |
| Beispiel 2 | 62% |
| Beispiel 3 | 52% |

Tabelle 6

Durchschnittsatemdruckanstieg von vier Meerschweinchen in % des Ausgangswertes nach Injizierung von 500 mg der erfindungsgemäß hergestellten Präparation pro kg Körpermasse

| | |
|---|---|
| Beispiel 1 | 102% |
| Beispiel 2 | 110% |
| Beispiel 3 | 125% |

Die Überlegenheit der erfindungsgemäß erhältlichen, intravenös anwendbaren Immunglobulin-G-hältigen Fraktionen gegenüber den bekannten, intramuskulär anwendbaren ist mit großer Anschaulichkeit aus den beiliegenden Diagrammen II bis IV zu ersehen.

Im Diagramm II sind die Blutdruckkurven bei systolischer und diastolischer Messung von jeweils vier Hunden aufgezeichnet, wobei auf der Abszisse jeweils die verabreichten Mengen in mg/kg Körpermasse der Tiere aufgetragen sind. Die voll ausgezeichnete Linie im Bereich von A bis B entspricht dem Verlauf des Blutdruckes bei Verabreichung von erfindungsgemäßen, intravenös anwendbaren Immunglobulin-G-hältigen Präparaten; der Verlauf der Kurve von A bis C (voll ausgezogene Linie und strichlierte Linie) entspricht dem Verlauf der Blutdruckkurve bei Applikation der im.-Standardsubstanz. Es ist ersichtlich, daß bei einer Applikation der im. anwendbaren Standardsubstanz von 5 mg/kg Körpermasse der Blutdruck um 30% abgefallen ist, wogegen bei der Applikation des erfindungsgemäßen Präparates dieser Abfall erst bei einer Dosierung von 500 mg/kg Körpermasse auftritt, d.h. das erfindungsgemäße intravenös anwendbare Immunglobulin hat eine um das mindestens 100fach geringere vasoaktive Wirkung als die bekannten im. anwendbaren Präparate unter sonst gleichen Bedingungen.

Aus dem Diagramm III ist die leukopenische Wirkung im Hundetest als Durchschnitt von vier Tieren im Vergleich zwischen dem erfindungsgemäßen, iv. anwendbaren Präparat und dem standardisierten, im. anwendbaren Standardpräparat zu ersehen. Die voll ausgezogene Linie im Bereich von A bis B entspricht dem erfindungsgemäß hergestellten intravenös anwendbaren Immunglobulin-G-hältigen Präparat, und die Linie von A bis C (voll ausgezogene Linie und strichlierte Linie) dem Verlauf der Leukozytenzahl bei Applikation der im. anwendbaren Standardsubstanz. Es ist ersichtlich, daß bei einer Applikation der im. anwendbaren Standardsubstanz die Leukozyten um 50% abgefallen sind, wogegen bei der Applikation des erfindungsgemäß hergestellten Präparates dieser Abfall erst bei einer Dosierung von 500 mg/kg Körpermasse auftritt, d.h. das erfindungsgemäß hergestellte, intravenös anwendbare Immunglobulin-G-hältige Präparat hat eine um das mindestens 1000 fach niedrigere leukopenische Wirkung als die bekannten, im. anwendbaren Präparate unter sonst gleichen Bedingungen.

Ähnlich sieht der Vergleich der bronchospastischen Wirkungen im Meerschweinchentest gemäß dem Diagramm IV aus, wobei der Verlauf der Kurve von A bis B (voll ausgezogene Linie) dem erfindungsgemäß hergestellten, iv. anwendbaren Präparat und der Verlauf der Kurve von A bis C (voll ausgezogene und strichlierte Linie) dem standardisierten im. anwendbaren Präparat entspricht. Der 30 %ige Atemdruckanstieg erfolgt beim bekannten im. anwendbaren Präparat schon bei einer Dosierung von 2 mg/kg Körpermasse, wogegen der gleiche Anstieg beim iv. anwendbaren Präparat erst bei einer Dosis von 500 mg/kg Körpermasse erfolgt, d.h. daß die bronchospastische Nebenwirkung bei dem erfindungsgemäßen Präparat um das 250fache geringer ist.

Aufgrund ihrer chemischen Zusammensetzung und ihrer pharmakologischen Eigenschaften sind die erfindungsgemäß erhältlichen Immunglobulin-G-hältigen Fraktionen in hervorragender Weise zur Verwendung für die Behandlung von primären und sekundären Immundefekten, A- oder Hypogammaglobulinamie, bei Antikörpermangelsyndrom, bei Virusinfektionen oder bakteriellen Infektionen sowie bei Autoimmun- und Immunkomplexverkrankungen geeignet.

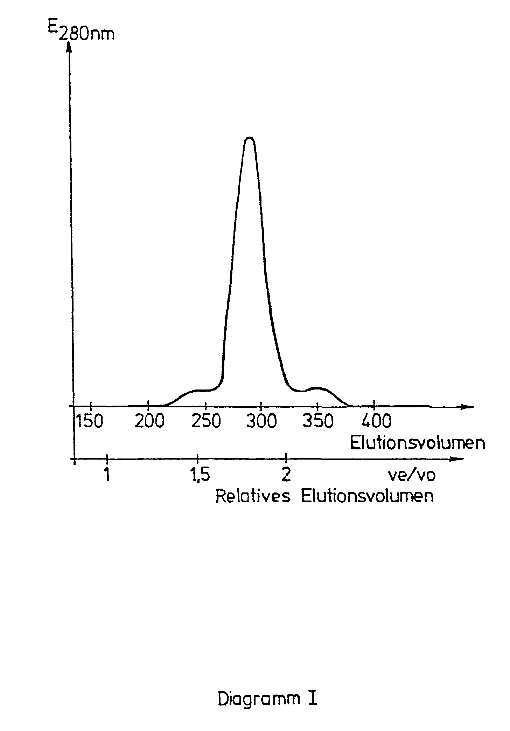

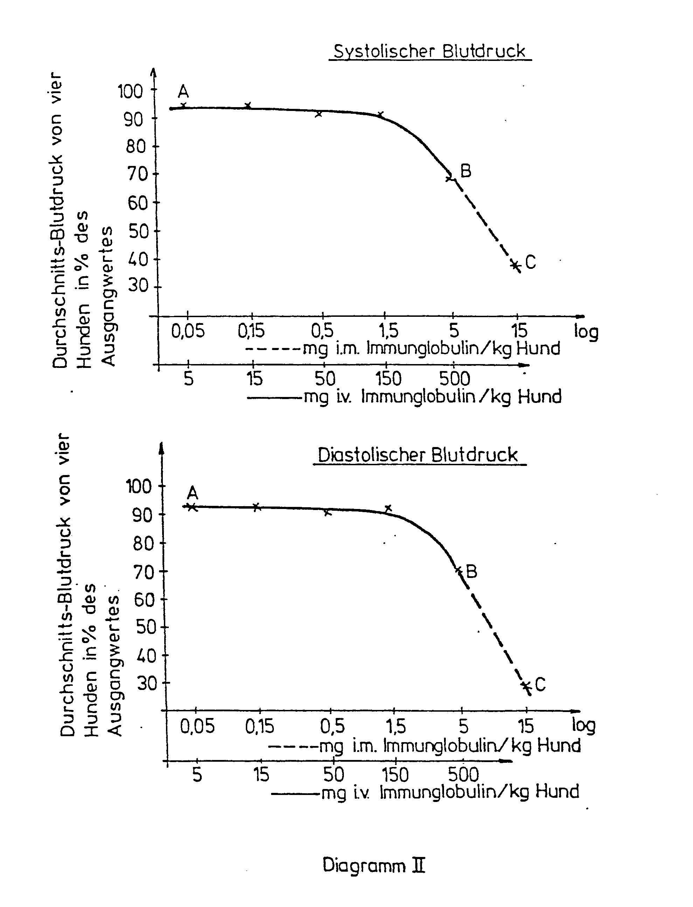

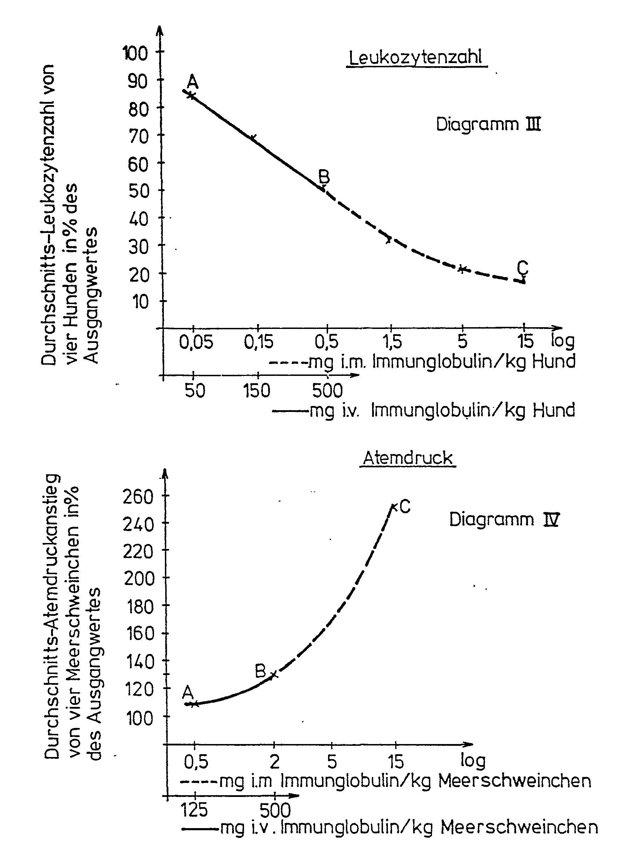

## Patentansprüche

1. Verfahren zur Inaktivierung von Unverträglichkeitsreaktionen verursachenden Substanzen in therapeutisch oder prophylaktisch anzuwendenden immunglobulinhältigen Blutfraktionen unter Verwendung von proteolytisch wirksamen Enzymen, dadurch gekennzeichnet, daß eine aus menschlichem oder tierischem Blut gewonnene immunglobulinhältige Blutfraktion mit an wasserunlösliches

8

Trägermaterial gebundenen Pankreasenzymen, wie Trypsin oder Chymotrypsin oder Pankreasprotease, behandelt wird, um enthaltene unerwünschte Substanzen zu inaktivieren, das immobilisierte Enzym abgetrennt und die behandelte Fraktion gegebenenfalls einer weiteren Fraktionierung und Konzentrierung unterworfen wird.

2. Verfahren nach Anspruch 1, dadurch gekennzeichnet, daß als wasserunlösliches Trägermaterial Sepharose 4 B-Gel® verwendet wird.

3. Verfahren nach Anspruch 1, dadurch gekennzeichnet, daß aus der mit an wasserunlösliches Trägermaterial gebundenen Enzymen behandelten immunglobulinhältigen Fraktion, gegebenenfalls nach Entfernen unerwünschter Begleitstoffe, das gereinigte Immunglobulin mit Proteinfällungsmitteln ausgefällt und zu einem Endprodukt verarbeitet wird.

4. Verfahren zur Herstellung einer immunglobulinhältigen Fraktion unter Anwendung des Verfahrens nach den Ansprüchen 1 bis 3, welche Fraktion mindestens 90% funktionell intakte IgG-Moleküle enthalten und im wesentlichen frei sind von vasoaktiv und leukopenisch wirksamen sowie von bronchospastischen Substanzen, ausgedrückt durch folgende Merkmale:

a) daß die vasoaktive Wirkung im Hundetest als Durchschnitt bei vier Tieren, d.h. ein Blutdruckabfall von höchstens 30%, erst bei einer Dosis von mehr als 500 mg/kg Körpermasse,

b) daß die leukopenische Wirkung im Hundetest als Durchschnitt bei vier Tierren, d.h. ein Abfall der Leukozytenzahl von höchstens 50%, erst bei einer Dosis von mehr als 500 mg/kg Körpermasse, und

c) daß die bronchospastische Wirkung im Meerschweinchentest als Durchschnitt bei vier Tieren, d.h. ein Atemdruckanstieg von höchstens 30%, erst bei einer Dosis von mehr als 500 mg/kg Körpermasse feststellbar ist, gekennzeichnet durch die Kombination der folgenden Maßnahmen,

daß aus menschlichem oder tierischem Plasma durch Behandeln mit Äthanol bei einer Temperatur von unter 0°C ein immunglobulinhältiger Niederschlag ausgefällt wird;

daß der Niederschlag mittels einer Pufferlösung extrahiert und aus der erhaltenen Lösung durch neuerliche Behandlung mit Äthanol ein pastenförmiges Immunglobulinkonzentrat gewonnen wird;

daß dieses Konzentrat durch Dialyse gereinigt wird;

daß die so gereinigte immunglobulinhältige Fraktion mit einem immobilisierten Enzym aus der Gruppe Trypsin, Chymotrypsin oder Pankreasprotease bei erhöhter Temperatur von etwa 37°C behandelt und sodann das immobilisierte Enzym abgetrennt wird;

daß aus der so behandelten Fraktion gereinigtes, im wesentlichen aus IgG bestehendes Immunglobulin mittels eines Proteinfällungsmittels, vorzugsweise Polyäthylenglykol, ausgefällt wird und daß die Fällung gelöst, die Lösung sterilfiltriert und schließlich lyophilisiert wird.

5. Verfahren zum Bestimmen von Unverträglichkeitsreaktionen verursachenden vasoaktiven Substanzen in therapeutisch und prophylaktisch anzuwendenden Blutprodukten, dadurch gekennzeichnet, daß die Blutprodukte intraarteriell Hunden injiziert werden und der Blutdruckabfall festgestellt wird.

6. Verfahren zum Bestimmen von Unverträglichkeitsreaktionen verursachenden leukopenischen Substanzen in therapeutisch und prophylaktisch anzuwendenden Blutprodukten, dadurch gekennzeichnet, daß die Blutprodukte intraarteriell Hunden injiziert werden und der Abfall der Leukozytenzahl festgestellt wird.

7. Verfahren zum Bestimmen von Unverträglichkeitsreaktionen verursachenden bronchospastischen Substanzen in therapeutisch und prophylaktisch anzuwendenden Blutprodukten, dadurch gekennzeichnet, daß die Blutprodukte intraarteriell Meerschweinchen injiziert werden und der Atemdruckanstieg festgestellt wird.

8. Verfahren nach den Ansprüchen 5 bis 7, dadurch gekennzeichnet, daß immunglobulinhältige Blutprodukte für die Bestimmung verwendet werden.

9. Verwendung des Verfahrens nach den Ansprüchen 5 bis 8 zur Prüfung der Sicherheit von immunglobulinhältigen Blutprodukten, mit der Maßgabe, daß für eine ausreichende Sicherheit bei maximal möglicher Dosierung ein signifikanter Abfall des Blutdruckes im Hundetest und/oder ein signifikanter Abfall der Leukozytenzahl im Hundetest und/oder ein signifikanter Atemdruckanstieg im Meerschweinchentest nicht feststellbar ist.

10. Verwendung des Verfahrens nach den Ansprüchen 5 bis 8 zur Prüfung der Sicherheit von immunglobulinhältigen Blutprodukten gemäß Anspruch 8, mit der Maßgabe, daß im Test auf vasoaktive Wirkung bei einer Dosis von mehr als 500 mg/kg Körpermasse im Durchschnitt bei vier Tieren ein Blutdruckabfall von höchstens 30% feststellbar ist.

11. Verwendung des Verfahrens nach den Ansprüchen 5 bis 8 zur Prüfung der Sicherheit von immunglobulinhältigen Blutprodukten gemäß Anspruch 8, mit der Maßgabe, daß im Test auf leukopenische Wirkung bei einer Dosis von mehr als 500 mg/kg Körpermasse im Durchschnitt bei vier Tieren ein Abfall der Leukozytenzahl von höchstens 50% feststellbar ist.

12. Verwendung des Verfahrens nach den Ansprüchen 5 bis 8 zur Prüfung der Sicherheit von immunglobulinhältigen Blutprodukten gemäß Anspruch 8, mit der Maßgabe, daß im Test auf bronchospastische Wirkung bei einer Dosis von mehr als 500 mg/kg Körpermasse im Durchschnitt bei vier Tieren ein Atemdruckanstieg von höchstens 30% feststellbar ist.

# EP  0 120 835  B1

**Revendications**

1. Procédé pour l'inactivation de substances provoquant des réactions d'incompatibilité dans des fractions du sang contenant une immunoglobuline à utiliser en thérapeutique ou en prophylaxie avec utilisation d'enzymes protéolytiques, caractérisé en ce que l'on traite une fraction de sang contenant de l'immunoglobuline, obtenue à partir de sang humain ou animal, avec des enzymes pancréatiques telles que trypsine ou chymotrypsine ou protéase pancréatique, fixées sur un support insoluble dans l'eau, pour inactiver les substances indésirables qu'elle contient, on sépare l'enzyme immobilisée et on soumet éventuellement la fraction traitée à un autre fractionnement et on la concentre.

2. Procédé selon la revendication 1, caractérisé en ce que l'on utilise comme matière de support insoluble dans l'eau le Sépharose 4 B Gel®.

3. Procédé selon la revendication 1, caractérisé en ce que l'immunoglobuline purifiée est précipitée par des agents de précipitation des protéines à partir de la fraction contenant l'immunoglobuline traitée par des enzymes fixées sur une matière de support insoluble dans l'eau, éventuellement après séparation des impuretés indésirables, et transformée en un produit final.

4. Procédé pour la fabrication d'une fraction contenant l'immunoglobuline par application du procédé selon les revendications 1 à 3, ladite fraction contenant au moins 90 moles % de molécules d'IgG fonctionnellement intactes et étant pratiquement exempte de substances vasoactives et à action leucopénique et de substances bronchospasmodiques, ce qui se manifeste par les caractères suivants:

a) l'activité vasoactive dans le test sur les chiens en moyenne sur quatre animaux, c-est-à-dire une chute de pression sanguine de 30% au plus, n'est décelable qu'à une dose de plus de 500 mg/kg de poids corporel,

b) l'action leucopénique dans le test sur les chiens en moyenne sur quatre animaux, c'est-à-dire une chute du nombre de leucocytes de 50% au plus, n'est décelable qu'à une dose de plus de 500 mg/kg de poids corporel et

c) l'action bronchospasmodique dans le test sur les cobayes en moyenne sur quatre animaux, c'est-à-dire une élévation de la pression respiratoire de 30% au plus, n'est décelable qu'à une dose de plus de 500 mg/kg de poids corporel, caractérisé par la combinaison des mesures suivantes:

on précipite un précipité contenant l'immunoglobuline à partir de plasma humain ou animal par traitement par l'éthanol à une température de moins de 0°C;

on extrait le précipité avec une solution tampon et on récupère par un nouveau traitement par l'éthanol un concentré pâteaux d'immunoglobuline de la solution obtenue;

on purifie ce concentré par dialyse;

on traite la fraction contenant l'immunoglobuline ainsi purifiée par une enzyme immobilisée choisie parmi la trypsine, la chymotrypsine et la protéase pancréatique à une température élevée d'environ 37°C et on sépare ensuite l'enzyme immobilisée;

on précipite à partir de la fraction ainsi traitée une immunoglobuline purifiée, consistant essentiellement en IgG, au moyen d'un agent de précipitation des protéines, de préférence le polyéthylèneglycol; et

on dissout le précipité, on filtre la solution en conditions stériles et enfin ou la lyophilise.

5. Procédé pour la détermination de substances vasoactives provoquant des réactions d'incompatibilité dans des produits du sang à appliquer en thérapeutique et en prophylaxie, caractérisé en ce que l'on injecte les produits du sang par voie intra-artérielle à des chiens et on détermine la chute de pression sanguine.

6. Procédé pour la détermination de substances leucopéniques provoquant des réactions d'incompatibilité dans des produits du sang à appliquer en thérapeutique et en prophylaxie, caractérisé en ce que l'on injecte les produits du sang par voi intra-artérielle à des chiens et on détermine la chute du nombre de leucocytes.

7. Procédé pour déterminer des substances bronchospasmodiques provoquant des réactions d'incompatibilité dans des produits du sang à appliquer en thérapeutique et en prophylaxie, caractérisé en ce que l'on injecte les produits du sang par voie intra-artérielle à des cobayes et on détermine l'élévation de la pression respiratoire.

8. Procédé selon les revendications 5 à 7, caractérisé en ce que l'on utilise pour la détermination des produits du sang contenant de l'immunoglobuline.

9. Utilisation du procédé selon les revendications 5 à 8, pour vérifier la sécurité de produits du sang contenant de l'immunoglobuline, avec la condition que, pour une sécurité suffisante à la dose maximale possible, on ne peut pas déceler une chute notable de la pression sanguine dans le test sur le chien et/ou une chute notable du nombre de leucocytes dans le test sur le chien et/ou une élévation notable de la pression respiratoire dans le test sur le cobaye.

10. Utilisation du procédé selon les revendications 5 à 8, pour vérifier la sécurité de produits de sang contenant de l'immunoglobuline selon la revendication 8, avec la condition que dans le test de l'action vasoactive, une chute de pression sanguine de 30% au plus en moyenne sur quatre animaux est décelable à une dose de plus de 500 mg/kg de poids corporel.

11. Utilisation du procédé selon les revendications 5 à 8, pour vérifier la sécurité de produits du sang contenant de l'immunoglobuline selon la revendication 8, avec la condition que dans le test de l'action

10

leucopénique, une chute du nombre de leucocytes de 50% au plus en moyenne sur quatre animaux est décelable à une dose de plus de 500 mg/kg de poids corporel.

12. Utilisation du procédé selon les revendications 5 à 8, pour vérifier la sécurité de produits du sang contenant de l'immunoglobuline selon la revendication 8, avec la condition que dans le test de l'action bronchospasmodique, une élévation de la pression respiratoire de 30% au plus en moyenne sur quatre animaux est décelable à une dose de plus de 500 mg/kg de poids corporel.

**Claims**

1. Method of inactivating incompatibility-reaction-causing substances in therapeutically or prophylactically applicable immunoglobulin-containing blood fractions while using proteolytically active enzymes, characterised in that an immunoglobulin-containing blood fraction obtained from human or animal blood is treated with pancreas enzymes, such as trypsin or chromotrypsin or pancreas protease, bound to water insoluble carrier material, so as to inactivate undesired substances contained therein, the immobilised enzyme is separated and the treated fraction optionally is subjected to a further fractionation and concentration.

2. Method according to claim 1, characterised in that Sepharose 4 B-gel® is used as the water insoluble carrier material.

3. Method according to claim 1, characterised in that the purified immunoglobulin is precipitated with the help of protein precipitating agents from the immunoglobulin-containing fraction treated with enzymes bound to water insoluble carrier material, if desired after removal of undesired accompanying substances, and is processed to obtain a final product.

4. Method of producing an immunoglobulin-containing fraction using the method according to claims 1 to 3, which fraction contains at least 90% functionally intact IgG molecules and is substantially free from vasoactive and leucopenically active as well as from bronchospastic substances, expressed by the following features:

a) that the vasoactive effect in the dog test as an average for four animals, i.e. a blood pressure decrease of at most 30%, is only detectable at a dose of more than 500 mg/kg body weight,

b) that the leucopenic effect in the dog test as an average of four animals, i.e. a decrease in the number of leucocytes of at most 50%, is only detectable at a dose of more than 500 mg/kg body weight, and

c) that the bronchospastic effect in the guinea pig test as an average of four animals, i.e. an increase in respiratory pressure of at most 30%, is detectable only at a dose of more than 500 mg/kg body weight, characterised by the combination of the following features,

that an immunoglobulin-containing precipitate is precipitated from human or animal plasma by treatment with ethanol at a temperature of below 0°C;

that the precipitate is extracted by means of a buffer solution and that a paste-like immunoglobulin concentrate is recovered from the solution obtained by further treatment with ethanol;

that this concentrate is purified by dialysis;

that the thus purified immunoglobulin-containing fraction is treated with an immobilised enzyme from the group trypsin, chymotrypsin or pancreas protease at an elevated temperature of about 37°C and then the immobilised enzyme is separated;

that, from the thus treated fraction, purified immunoglobulin, substantially consisting of IgG, is precipitated by means of a protein precipitating agent, preferably polyethylene glycol, and

that the precipitate is dissolved, the solution sterile filtered and finally lyophilised.

5. Method of determining incompatibility-reaction-causing vasoactive substances in therapeutically and prophylactically applicable blood products, characterised in that the blood products are intraarterially injected in dogs and that the blood pressure decrease is determined.

6. Method of determining incompatibility-reaction-causing leucopenic substances in therapeutically and prophylactically applicable blood products, characterised in that the blood products are intraarterially injected in dogs and that the decrease in the number of leucocytes is determined.

7. Method of determining incompatibility-reaction-causing bronchospastic substances in therapeutically and prophylactically applicable blood products, characterised in that the blood products are intraarterially injected in guinea pigs and that the increase in respiratory pressure is determined.

8. Method according to claims 5 to 7, characterised in that immunoglobulin-containing blood products are used for the determination.

9. Use of the method according to claims 5 to 8 for testing the safety of immunoglobulin-containing blood products, with the provision that for a sufficient safety with a maximumly possible dose a significant decrease in blood pressure in the dog test and/or a significant decrease in the number of leucocytes in the dog test and/or a significant increase in the respiratory pressure in the guinea pig test is not detectable.

10. Use of the method according to claims 5 to 8 for testing the safety of immunoglobulin-containing blood products according to claim 8, with the provision that in the test for vasoactive effect at a dose of more than 500 mg/kg body weight a decrease in blood pressure of at most 30% is detectable as an average of four animals.

11. Use of the method according to claims 5 to 8 for testing the safety of immunoglobulin-containing blood products according to claim 8, with the provision that in the test for leucopenic effect at a dose of

11

more than 500 mg/kg body weight a decrease in the number of leucocytes of at most 50% is detectable as an average of four animals.

12. Use of the method according to claims 5 to 8 for testing the safety of immunoglobulin-containing blood products according to claim 8, with the provision that in the test for bronchospastic effect at a dose of more than 500 mg/kg body weight an increase in respiratory pressure of at most 30% is detectable as an average of four animals.

Diagramm I

Diagramm II

2

Leukozytenzahl

Diagramm III

Durchschnitts–Leukozytenzahl von vier Hunden in% des Ausgangwertes

100
90
80
70
60
50
40
30
20
10

A
B
C

0,05   0,15   0,5   1,5   5   15   log
----mg i.m. Immunglobulin/kg Hund

50   150   500
—mg i.v. Immunglobulin/kg Hund

Atemdruck

Diagramm IV

Durchschnitts–Atemdruckanstieg von vier Meerschweinchen in% des Ausgangwertes

260
240
220
200
180
160
140
120
100

A
B
C

0,5   2   5   15   log
----mg i.m Immunglobulin/kg Meerschweinchen

125   500
—mg i.v. Immunglobulin/kg Meerschweinchen